(19) Europäisches Patentamt
European Patent Office
Office européen des brevets

(11) EP 0 867 695 A2

(12) # EUROPÄISCHE PATENTANMELDUNG

(43) Veröffentlichungstag:
**30.09.1998 Patentblatt 1998/40**

(51) Int. Cl.[6]: **G01F 23/24**

(21) Anmeldenummer: **98105391.1**

(22) Anmeldetag: **25.03.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **26.03.1997 DE 19712577**
**10.04.1997 DE 19714870**

(71) Anmelder:
**Bilfinger + Berger Bauaktiengesellschaft**
**68165 Mannheim (DE)**

(72) Erfinder:
- **Brosig, Stefan, Dr.**
  **70 563 Stuttgart (DE)**
- **Gläser, Eberhard**
  **73773 Aichwald 4 (DE)**

(54) **Verfahren und Vorrichtung zur Feststellung der Verfüllung von Schalungen mit Beton sowie von dessen Verdichtung unter Ausnutzung seiner elektrischen Eigenschaften**

(57) Die Erfindung betrifft ein Verfahren und dazugehörige Vorrichtungen, mit dem sehr kostengünstig und dennoch zuverlässig festgestellt werden kann, ob Schalungen, vor allem solche, an denen keine Sichtfenster möglich sind, vollständig mit Beton gefüllt sind. Es beruht auf der Messung der elektrischen Größen Leitfähigkeit, bzw. Widerstand (Gleich- oder Wechselstromwiderstand) in den kritischen Bereichen der Schalung (isolierende Luft wird durch den aufgrund seiner Alkalinität leitfähigen Beton ersetzt).
Alternativ zur einfachen Leitfähigkeitsmessung ist auch eine Messung der Änderung der Kapazität eines einfachen Kondensators nach Prinzip des Plattenkondensators möglich, wenn dessen Dielektrikum beim Füllvorgang durch Beton ersetzt wird.
Es ist auch gleichzeitig möglich, die Verdichtung des Betons über zeitabhängige Messungen der von der Dichte des Betons abhängigen elektrischen Größen Leitfähigkeit, bzw. Widerstand (Gleich- oder Wechselstromwiderstand), oder Permittivität zu bestimmen.
Die Messung der Änderung der Permittivitätszahl $\varepsilon_R$ kann z.B. über die Änderung der Kapazität eines einfachen Kondensators nach Prinzip des Plattenkondensators erfolgen.
Wegen ihres geringen Preises können die Meßsonden, im einfachsten Falle der Leitfähigkeits- oder Widerstandsmessung einfache Kabel, bei kapazitiver Messung isolierte Platten, im Beton verbleiben (die Kabel werden dann einfach abgeschnitten), ein Herausziehen ist jedoch auch möglich. Das Verfahren ist einfach und robust, es ist für seine Anwendung kein Ingenieur erforderlich.

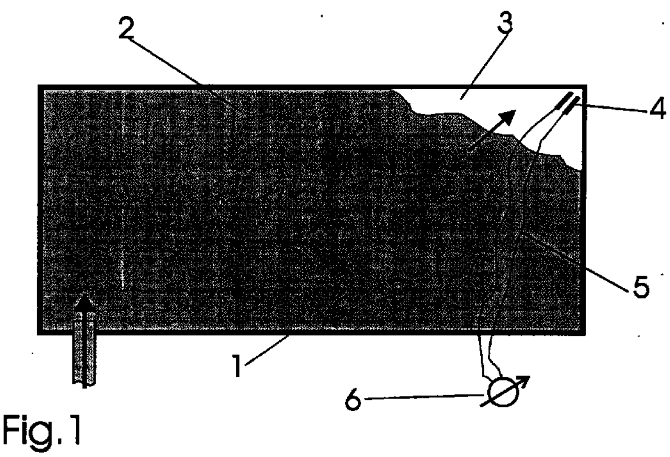


Fig. 1



Printed by Xerox (UK) Business Services
2.16.3/3.4

**Beschreibung**

Im Bauwesen tritt beim Füllen einer Schalung mit Beton oft das Problem auf, daß geprüft werden muß, ob die Schalung vollständig mit Beton verfüllt ist, denn ein übermäßiges Einpressen von Beton kann die zum Teil teuren Schalungen zerstören. Nicht immer ist es möglich, Sichtfenster an den kritischen Stellen der Schalung anzubringen. Vor allem im Tunnelbau tritt dieses Problem verstärkt auf (z.B. am First des Deckengewölbes).
Daher besteht weiterhin der Bedarf nach zuverlässigen und bautauglichen Verfahren für die Kontrolle des Verfüllungsgrades von Schalungen. Weiterhin tritt im Bauwesen beim Füllen einer Schalung mit Beton oft das Problem der Dichtemessung am flüssigen Beton auf. Beim Verdichten desselben wird die in ihm befindliche leichte Luft herausgedrängt und seine Dichte steigt an. Bislang ist es allerdings nicht möglich „in vivo" (im Bauteil selbst) die Dichte zu messen. Es müssen Erfahrungswerte herangezogen werden. Dennoch tritt immer wieder das Problem einer zu geringen Verdichtung auf mit daraus folgender mangelhafter Festigkeit des erhärteten Betons. Störend ist auch der schlechte optische Eindruck an der Oberfläche und die höhere Korrosionsanfälligkeit von Bewehrungen in schlecht verdichtetem Beton. Nicht immer ist auch eine optische Kontrolle wenigstens der Oberfläche möglich.Vor allem im Tunnelbau tritt dieses Problem verstärkt auf. Daher besteht auch weiterhin der Bedarf nach zuverlässigen und bautauglichen Verfahren für die Messung der Betonverdichtung.
Erfindungsgemäß wird daher vorgeschlagen, die Verfüllung von Schalungen und, wenn nötig, gleichzeitig die Betonverdichtung unter Ausnutzung der elektrischen Eigenschaften des Betons zu bestimmen.

Die Erfindung sieht im einfachsten Falle vor, durch Messung der elektrischen Leitfähigkeit in den kritischen Bereichen der Schalung festzustellen, ob dort die elektrisch isolierende Luft durch den aufgrund seiner Alkalinität leitfähigen flüssigen Beton ersetzt wird. (Das Verfahren ist dabei aber nicht auf Beton als Bindemittel beschränkt, da auch andere nichtalkalische Bundemittel gegenüber der Luft stark erhöhte Leitfähigkeit aufweisen).
Beton weist aufgrund seiner hohen Alkalinität eine hohe Konzentration an Hydroxidionen auf, die trotz seiner hohen Viskosität eine erhebliche elektrische Leitfähigkeit bedingen. Grund hierfür ist der sogenannte Grotthus-Mechanismus der Ionenwanderung für Hydroxid-Ionen in einem elekrtischen Feld: Das Ion wandert dabei nicht stofflich, sondern es findet über viele über Wasserstoffbrückenbindungen (········) angebundene Wassermoleküle hinweg nur ein Umklappen von chemischen Bindungen statt:

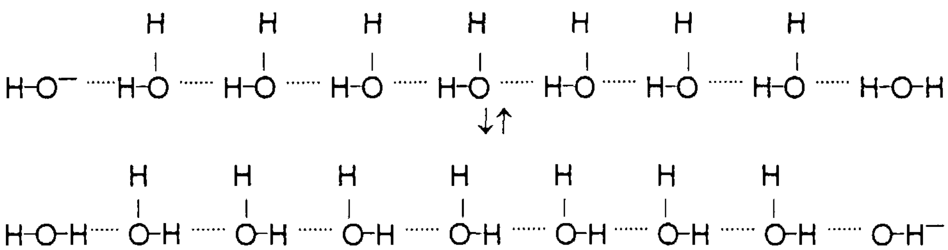

Bringt man (siehe hierzu Fig. 1) zwei beabstandete Elektroden (im einfachsten Falle z.B. nicht isolierte Drahtenden) in den kritischen Bereich der Schalung ein (Befestigung elektrisch isoliert an der Armierung, der Schalung oder mittels eines speziellen Trägers) und mißt zwischen diesen beiden Elektroden den elektrischen Widerstand (was stromlos erfolgen kann), so kann man den Zeitpunkt der Verfüllung der Schalung zuverlässig am plötzlichen starken Abfall des elektrischen Widerstandes feststellen. Äquivalent kann auch die Zunahme der Leitfähigkeit zwischen den beiden Drahtenden gemessen werden. Bevorzugt geschieht dies dann mit Wechselstrom, so daß keine Elektrolyseerscheinungen auftreten. Es kann auch eine pulsförmige Spannungsquelle eingesetzt werden.
Die beiden Elektroden haben dabei bevorzugt einen Abstand, der so groß ist, daß kein Kondenswasser oder spritzender Beton einen Kurzschluß zwischen den beiden Elektroden hervorrufen kann, der eine Verfüllung vortäuschen würde; der Abstand kann im Bereich von Millimetern bis Dezimetern liegen, bei größeren Schalungen ist ein Abstand von größenordnungsmäßig 5 bis 10 Zentimetern sinnvoll.
Je größer die Fläche der Elektroden ist, und je geringer ihr Abstand voneinander, umso unempfindlicher (und billiger) können die Meßgeräte sein. Der elektrische Widerstand wird an einem einfachen Leitfähigkeits- oder Widerstandsmeßgerät überwacht; es ist auch ein Gerät möglich, welches akkustisch oder optisch Meldung macht. Ein elektrischer Schreiber ist ebensomöglich wie auch eine Verarbeitung der Daten über einen Rechner.
Es besteht die Möglichkeit, mit nur einem Kabelpaar mehrere Stellen der Schalung auf Verfüllung zu überwachen: Hierzu weist das Kabelpaar entlang seiner Länge paarweise Elektroden auf, die voneinander beabstandet sind (siehe Fig.2a). Bei Verfüllung mit Beton werden diese paarweisen freiliegenden Elektroden nacheinander kurzgeschlossen, wobei durch die gegenüber dem Metalldraht geringere Leitfähigkeit des Betons ein stufenweiser Rückgang des elektrischen Widerstandes mit jeder kurzgeschlossenen nichtisolierten Stelle erfolgt (bzw. ein stufenweises Ansteigen der Leitfähigkeit, siehe Fig.2b). Damit kann an einem Meßgerät verfolgt werden, wie weit der Beton die Schalung bereits verfüllt hat und die Einpreßgeschwindigkeit dem Verfüllungsgrad angepaßt werden.

Es ist auch möglich, statt mit zwei Drähten mit nur einem Draht auszukommen: in diesem Fall wird eine Zuleitung des Meßgerätes geerdet und man mißt die plötzliche Abnahme des Widerstandes über Beton und Boden, wenn die isolierende Luft verdrängt ist. Ein solcher Kurzschluß ist vergleichbar den Elektozäunen bei der Vieh- und Pferdehaltung, bei denen der Kontakt des Tieres mit dem elektrisch leitfähigen Zaun den Kurzschluß zum Erdreich bewirkt.
Auch hier kann man bei Verwendung eines Drahtes mit Elektroden in Reihe, (z.B. mehreren abisolierten Stellen eines Drahtes) einen stufenweisen Zusammenbruch des elektrischen Widerstandes (bzw. ein stufenweises Zunehmen der Leitfähigkeit) messen und damit den Verfüllungsgrad verfolgen. Die Elektroden können auch an Verzweigungen des ursprünglichen Drahtes angebracht sein, es sind sogar regelrechte baumartige Verzweigungsstrukturen denkbar (siehe Fig.5). Ähnliche Abzweigungen sind auch für mehrere paarweise angeordnete Elektroden möglich, wobei darauf geachtet werden muß, daß die eine Elektrode eines Elektrodenpaares zum Draht der einen Polarität Verbindung hat, die andere zu dem der anderen Polarität.
Meßdrähte können in dem fertigen Beton verbleiben, sie können aber nach Verfüllung und vor Aushärtung auch sehr einfach gezogen und wiederverwendet werden.
Statt einfachen freiliegenden Drahtbereichen zylindrischer Form sind zur Vergrößerung der freiliegenden Metallflächen z.B. auch Blech-, Folien- oder Rohrform möglich.
Eine Verwendung von parallel zum Kabel liegenden Armierungseisen als Stromableitung ist denkbar.

Im vorhergehenden wurde ein einfaches Verfahren beschrieben, welches es erlaubt, die Verfüllung einer Schalung (selbstverständlich auch eines beliebigen anderen Hohlraumes) durch Messung der elektrischen Leitfähigkeit des Betons oder der dielektrischen Eigenschaften des Betons in den kritischen Bereichen der Schalung (bzw. des Hohlraumes) festzustellen. Dort wird die elektrisch isolierende Luft, die ein Dielektrikum mit der Permittivitätszahl (auch als relative Permittivität oder Dielektrizitätszahl bekannt) $\varepsilon_R \approx 1$ darstellt, durch den aufgrund seiner Alkalinität elektrisch leitfähigen flüssigen Beton ($\varepsilon_R$ größenordnungsmäßig 5 bis 20, je nach Wassergehalt und Zuschlagstoffen) ersetzt. (Das Verfahren ist dabei aber nicht auf Beton als Bindemittel beschränkt, da auch andere nichtalkalische Bindemittel gegenüber der Luft stark erhöhte Leitfähigkeit oder veränderte dielektrische Eigenschaften aufweisen).
Bei Unterwasserbetonierungen wird entsprechend Wasser (je nach Salzgehalt sehr schlecht oder gut leitend; $\varepsilon_R \approx 80$) durch Beton anderer Leitfähigkeit und Permittivität ersetzt.
Durch Vergleich mit Normwerten oder zeitabhängige Messungen der elektrischen Größen mit genormten Elektroden läßt sich dieses einfache Verfahren weiter verfeinern, so daß man über die Dichte des Betons und damit seine Qualität eine quantitative Antwort erhält.
Dabei werden „in vivo" gemessene Werte mit „in vitro" (z.B. im Labor) gemessenen Referenzwerten verglichen und daraus die Dichte vor Ort berechnet. Schwierigkeiten bereiten dabei aber vor allem die starke Abhängigkeit der Leitfähigkeit von der Temperatur, die das zusätzliche Vorhandensein eines Temperaturmeßfühlers erfordert, und von der Viskosität (abhängig von der Rüttelenergie am Ort der Messung!). Zuverlässiger ist daher die Permittivitätszahl als Meßgröße heranzuziehen. Die Permittivitätszahl $\varepsilon_R$ ist z.B. in den Gleichungen für die Kapazität von Kondensatoren enthalten, so daß Kapazitätsmessungen Aufschluß über ihre Größe geben.
(Kapazität C eines einfachen Plattenkondensators mit Platten des Abstandes d und der Fläche A:

$$C = \varepsilon_R \cdot \varepsilon_0 \cdot \frac{A}{d}$$

$\varepsilon_0$ elektrische Feldkonstante)
Die Permittivitätszahl weist eine geringere Temperaturabhängigkeit und fast gar keine Viskositätsabhängigkeit in ihrer Größe auf.
Dennoch sollte auch in diesem Falle von jedem Betonansatz vorher eine Probe genommen und „in vitro" durchgemessen werden, da die Betonzusammensetzung nie völlig konstant ist.
Um die beschriebenen Schwierigkeiten zu umgehen, schlägt eine bevorzugte Ausführung der Erfindung daher ein etwas verändertes Meßverfahren vor, bei dem nicht Absolutwerte der elektrischen Kenngrößen (Leitfähigkeit, bzw. Widerstand, oder Kapazität) gemessen werden, sondern deren relative Veränderung mit der Zeit. Dabei wird die Tatsache ausgenützt, daß das erstrebte Endziel beim Bau der völlig verdichtete Beton ist. Ist dieses Ziel erreicht, finden aber auch keine Veränderungen in der Umgebung der Meßsonden mehr statt. Damit bleiben dann auch die gemessenen elektrischen Kenngrößen konstant. Das Verfahren sieht somit vor, so lange Schwingungsenergie in den Beton einzubringen, bis die Meßgeräte keine oder nur noch geringfügige Veränderungen der gemessenen Größen anzeigen. Bei hinreichend geringen Änderungen kann ein optisches und/oder akkustisches Signal erfolgen, ähnlich, wie es bei modernen digitalen Fieberthermometern der Fall ist, die anfangen zu piepsen, wenn keine meßbare Temperaturänderung mehr stattfindet.

Gerade für Fälle, in denen es nicht auf höchste Qualität des Betons ankommt, ist aber auch noch eine gewisse, empirisch zu bestimmende, Veränderung der gemessenen Größen bei Abbruch des Eintragens von Schwingungsenergie tragbar.

Inhomogenitäten im Beton (z.B. Kieselsteine) erzeugen bei ihrem Durchgang zwischen den Elektroden Meßwertschwankungen.
Es gibt zwei prinzipielle Methoden, über die Inhomogenitäten des Betons (Grob- und Feinanteile) zu mitten: räumliche Mittelung und zeitliche Mittelung. Beide Methoden können auch kombiniert werden zu einer „raumzeitlichen" Mittelung. Bei der räumlichen Mittelung werden die Abmessungen der Elektroden groß gegenüber denen der Inhomogenitäten gewählt, bei der zeitlichen Mittelung wird über einen Zeitraum gemittelt, der groß ist gegenüber dem Zeitraum, den eine Inhomogenität benötigt, die Meßsonde zu passieren. Fig. 6 zeigt (am Beispiel der Permittivität) schematisch die Meßkurve der elektrischen Kenngröße bei räumlicher Mittelung (große Elektroden), Fig.7 (ebenfalls am Beispiel der Permittivität) die Meßkurve vor einer zeitlichen Mittelung (grau), und nach einer solchen Mittelung (Glättung der Kurve; schwarz). Die zeitliche Mittelung kann elektronisch oder durch ein träge reagierendes Meßgerät erfolgen.

Bevorzugt weisen die Elektroden eine Fläche auf, die ungefähr mindesten 5 mal so groß ist wie die senkrecht projizierte Fläche der größten üblichen Inhomogenität.
Für permittivitätsabhängige Messungen weist dabei eine Elektrode bevorzugt eine seitliche Ausdehnung von ungefähr 1 bis 30 Zentimetern auf, für leitfähigkeitsabhängige (widerstandsabhängige) Messungen von ungefähr 0,2Milimetern bis 30 Zentimetern.

Fig. 8 zeigt von der Seite den speziellen Fall eines Plattenkondensators, zwischen dessen Platten die Betonverdichtung vor sich geht. Der Abstand der Platten eines solchen Kondensators ist vorteilhafterweise ungefähr so groß (oder größer), wie der Durchmesser der größten typischen festen Inhomogenitäten im Beton. Dadurch wird ein Verstopfen des Durchganges zwischen den Platten vermieden. Ein Abstand von größenordnungsmäßig 2 Zentimetern (ungefähr 0,5 bis 5 Zentimetern) ist sinnvoll und wird durch isolierte oder isolierende Abstandshalter gewährleistet. Die Metallplatten oder -folien sind durch eine dünne Isolationsschicht (z.B. Kunststoff) gegen den Beton isoliert, um Spannungsverluste durch die Leitfähigkeit des Betons zu verhindern. In einer einfachen Ausführung reicht hierfür schon ein dichter Kunststoffbeutel, z.B. ein Gefrierbeutel, aus.
Bei Messung der Leitfähigkeit bzw. des Widerstandes ist ein ähnlicher plattenartiger Aufbau möglich, doch fehlt wenigstens ein Teil der Isolation. Elektroden können jedoch auch fast beliebige andere Formen aufweisen.

Ob bei Messung der Leitfähigkeit (bzw. des Widerstandes) oder der Permittivität: Die Meßsonden sind an der Bewehrung, an der Schalung, oder an speziellen Trägern ortsfest angebracht.
Es können mehrere Meßsonden unabhängig voneinander an verschiedenen Stellen der Schalung die Betonverdichtung messe. Dabei können die einzelnen Meßsonden über eigene Meßgeräte verfügen oder an einem gemeinsamen Meßgerät angeschlossen sein, das über Wechselschalter Verbindung zur jeweiligen Meßsonde aufnimmt.
Mit der Vorrichtung läßt sich nicht nur die Betonverdichtung messen, sondern auch die Verfüllung des Hohlraumes, wenn nämlich die Meßsonden an Stellen der Schalung plaziert sind, die als letzte von Beton erreicht werden. Eine drastische Änderung der elektrischen Meßgröße zeigt dabei an, daß der Beton die Meßsonde erreicht hat und umspült, eine langsame Änderung und Erreichen eines Grenzwertes ist Zeichen für die anschließend stattfindende Verdichtung.

Die Figuren stellen schematisch und nicht maßstäblich einige Ausführungsformen des Verfahrens dar. Die Befestigung für die Leitungen (z.B. an der Armierung, an der Schalung oder an einem speziellen Träger) ist nicht gezeichnet.

Fig.1 zeigt eine Schalung beim Verfüllen mit einem Elektrodenpaar als Meßsonde.

Fig.2a zeigt eine Schalung beim Verfüllen mit mehreren Elektrodenpaaren, die hintereinander angeordnet sind.

Fig.2b zeigt schematisch das stufenweise Anwachsen der Leitfähigkeit mit Erreichen jedes Elektrodenpaares durch den Beton, wie es z.B. bei Verwendung eines elektrischen Schreibers aufgezeichnet wird. Durch Abzählen der Stufen erkennt man, wieviele Elektrodenpaare bereits von Beton umhüllt sind.

Fig. 3 zeigt das Verfahren unter Verwendung nur einer Elektrode und Messung gegen die Erde.

Fig.4 zeigt das Verfahren unter Verwendung mehrerer hintereinander liegender und daher zeitlich nacheinander umflossener Elektroden und Messung gegen die Erde.

Fig.5 zeigt das Verfahren unter Verwendung baumartig verzweigter Elektrodenzuleitungen und Messung gegen die Erde.

Fig. 6 zeigt (am Beispiel der Permittivität) schematisch die Meßkurve der elektrischen Kenngröße bei räumlicher Mittelung (große Elektroden).

Fig.7 zeigt (ebenfalls am Beispiel der Permittivität) die Meßkurve vor einer zeitlichen Mittelung (grau), und nach einer solchen Mittelung (Glättung der Kurve; schwarz).

Fig. 8 zeigt von der Seite den speziellen Fall eines Plattenkondensators, zwischen dessen Platten die Betonverdichtung vor sich geht. Ein Abstand wird durch isolierte oder isolierende Abstandshalter gewährleistet. Die Metallplatten oder -folien sind durch eine dünne Isolationsschicht (z.B. Kunststoff) gegen den Beton isoliert.

Legende

1 Schalung
2 eingepreßter Beton
3 noch verbliebener Hohlraum
4 Elektrode
5 isoliertes Kabel
6 Meßgerät
7 Erdung
8 Kondensatorplatte
9 Isolationsschicht
10 isolierender, stabförmiger Abstandshalter

**Patentansprüche**

1. Verfahren zur Feststellung des Füllungsgrades von Schalungen mit Beton, dadurch gekennzeichnet, daß an mindestens einem Ort in der Schalung eine Meßsonde plaziert ist, die die Änderung der elektrischen Eigenschaften in ihrer näheren Umgebung mißt.

2. Verfahren nach Anspruch 1, dadurch gekennzeichnet, daß diese Messung an kritischen Punkten bei der Verfüllung der Schalung erfolgt, bevorsugt an den Orten, die als letzte im Füllvorgang vom Beton erreicht werden.

3. Verfahren nach mindestens einem der Ansprüche 1 und 2, dadurch gekennzeichnet, daß die Änderung des elektrischen Widerstandes oder der elektrischen Leitfähigkeit zwischen jeweils zwei beabstandeten Elektroden gemessen wird, wenn das diese ursprünglich umgebende Medium, meist Luft, durch Beton ersetzt wird.

4. Verfahren nach mindestens einem der Ansprüche 1 und 2, dadurch gekennzeichnet, daß die Änderung des elektrischen Widerstandes oder der elektrischen Leitfähigkeit mittels einer Elektrode gegen die Erdung gemessen wird, wenn das die Elektrode ursprünglich umgebende Medium, meist Luft, durch Beton ersetzt wird.

5. Verfahren nach mindestens einem der Ansprüche 1 bis 3, dadurch gekennzeichnet, daß mindestens 2 Paare von Elektroden so geschaltet werden, daß im Verlauf der Füllung der Schalung mit Beton erst das eine Paar von Elektroden umflossen wird und eine Leitfähigkeitszunahme bewirkt, dann das nächste Paar, usw.

6. Verfahren nach mindestens einem der Ansprüche 1, 2 und 4, dadurch gekennzeichnet, daß mindestens zwei Elektroden in Reihe angeordnet werden, die im Verlauf der Füllung zeitlich nacheinander umflossen werden, wodurch gegenüber der Erdung eine Leitfähigkeitszunahme bewirkt wird.

7. Verfahren nach mindestens einem der Ansprüche 1 bis 6, dadurch gekennzeichnet, daß die Elektroden durch Abisolierung von Bereichen eines Kabels hergestellt werden.

8. Verfahren nach mindestens einem der Ansprüche 1 bis2, dadurch gekennzeichnet, daß die Meßsonde als Plattenkondensatoren ausgeführt sind, deren Kapazitätsänderung bei Verdrängung des Dielektrikums Luft (bzw. Wasser bei Unterwasserbetonierungen) durch Beton gemessen wird.

9. Verfahren nach mindestens einem der Ansprüche 1 bis 8, dadurch gekennzeichnet, daß verzweigte Strukturen als Aufbau der Zuleitungen zu den Elektroden verwendet werden, die mit nur einem Meßgerät die Überwachung der Verfüllung an vielen Meßpunkten gestattet.

10. Verfahren nach Anspruch 9, dadurch gekennzeichnet, daß Elektrodenpaare oder Elektroden unterschiedlicher Größe und/oder Form verwendet werden, um auch aus der Größe der Änderung der elektrischen Kenngröße auf den Ort des eingebrachten Betons zu diesem Zeitpunkt schließen zu können.

**11.** Vorrichtung zur Durchführung des Verfahren nach mindestens einem der Ansprüche 1 bis 10,
dadurch gekennzeichnet, daß sie mindestens aus folgenden Bestandteilen besteht:

a.) Meßgerät für die elektrische Größe Widerstand, bzw. Leitfähigkeit, oder Kapazität
b.) mindestens einer Elektrode, wenn Widerstand oder Leitfähigkeit über die Erde gemessen wird, oder mindestens einem Elektrodenpaar, wenn der Widerstand, bzw. die Leitfähigkeit, oder die Kapazität zwischen den Elektroden gemessen wird
c.) isolierten Zuleitungskabeln zu den Elektroden.

**12.** Verfahren zur Feststellung der Verdichtung von Beton,
dadurch gekennzeichnet, daß an mindestens einem Ort im Beton eine Meßsonde plaziert ist, die

a.) entweder in Größe, Form, Fläche, Abstand genormt ist, die elektrischen Eigenschaften des Betons in ihrer näheren Umgebung mißt und diese mit einem Referenzsollwert vergleicht, oder
b.) ungenormt sein kann und dafür die Änderung der betreffenden elektrischen Eigenschaft mit der Zeit verfolgt.

**13.** Verfahren nach Anspruch 12,
dadurch gekennzeichnet, daß es sich bei den zur Messung der Betonverdichtung herangezogenen elektrischen Eigenschaften des Betons um seine elektrische Leitfähigkeit, bzw. seinen elektrischen Widerstand, oder die Permittivitätszahl handelt.

**14.** Verfahren nach mindestens einem der Ansprüche 12 und 13,
dadurch gekennzeichnet, daß der elektrische Widerstand oder die elektrische Leitfähigkeit zwischen zwei beabstandeten Elektroden gemessen wird, während die Betonverdichtung stattfindet.

**15.** Verfahren nach mindestens einem der Ansprüche 12 bis 14,
dadurch gekennzeichnet, daß der elektrische Widerstand, bzw. die elektrische Leitfähigkeit mittels einer Elektrode gegen die Erdung gemessen wird, während die Betonverdichtung stattfindet.

**16.** Verfahren nach Anspruch 12 und 13,
dadurch gekennzeichnet, daß die permittivitätsabhängige Kapazität eines Kondensators, bevorzugt eines Plattenkondensators, zur Bestimmung der Betonverdichtung herangezogen wird, indem wiederum von dieser abhängigen Größen, z.B. die Frequenz eines Schwingkreises aus der zu bestimmenden Kapazität und einer vorgegebenen Induktivität, gemessen werden.

**17.** Verfahren nach mindestens einem der Ansprüche 12 bis 15
dadurch gekennzeichnet, daß der elektrische Widerstand hochohmig oder stromlos, z.B. mittels einer Art Wheatstone-Brücke, gemessen wird.

**18.** Verfahren nach mindestens einem der Ansprüche 12 bis 15 und 17,
dadurch gekennzeichnet, daß die elektrische Leitfähigkeit, bzw. der elektrische Widerstand, mit Wechselstrom, Gleichstrom, oder gepulstem Strom gemessen wird.

**19.** Vorrichtung zur Durchführung des Verfahren nach mindestens einem der Ansprüche 12 bis 18
dadurch gekennzeichnet, daß sie mindestens aus folgenden Bestandteilen besteht:

a.) Meßgerät für die elektrische Größe Widerstand, bzw. Leitfähigkeit, oder Permittivität, welches in der Lage ist, die elektrischen kenngrößen oder davon abhängige Meßgrößen auf mindestens 20% genau, bevorzugt 5% genau oder besser, zu messen und anzuzeigen.
b.) mindestens einer Elektrode, wenn Widerstand oder Leitfähigkeit über die Erdung gemessen wird, oder mindestens einem Elektrodenpaar, wenn der Widerstand, bzw. die Leitfähigkeit, oder die Permittivität zwischen den Elektroden gemessen wird, wobei bei Messung der Permittivität, z.B. über die Kapazität, die Elektroden elektrisch gegen die Umgebung isoliert sind
c.) isolierten Zuleitungskabeln zu den Elektroden.

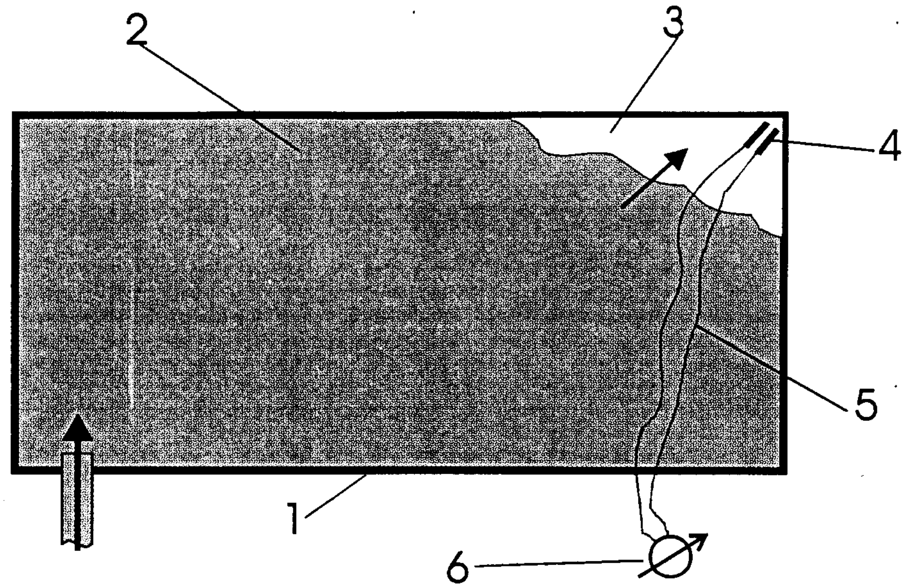


Fig.1

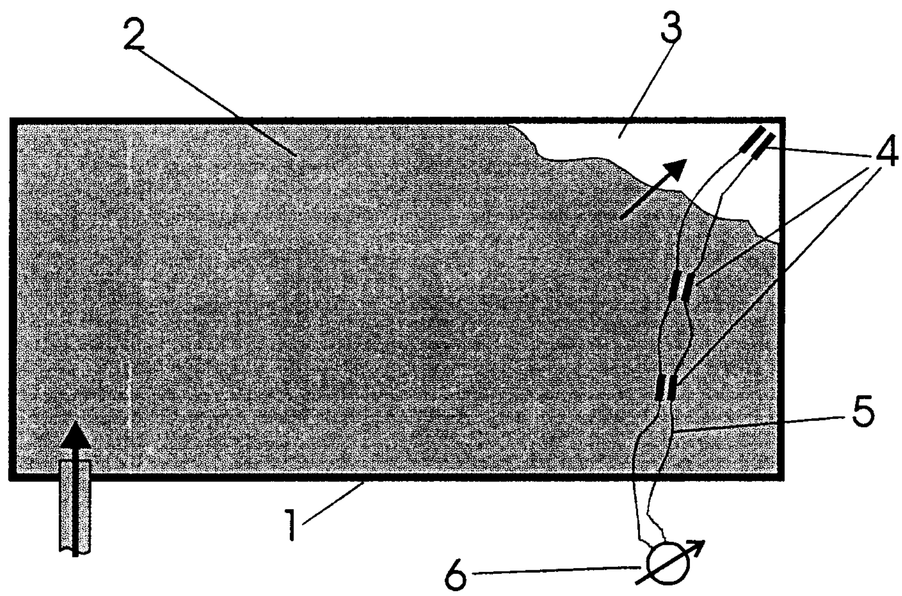


Fig.2a

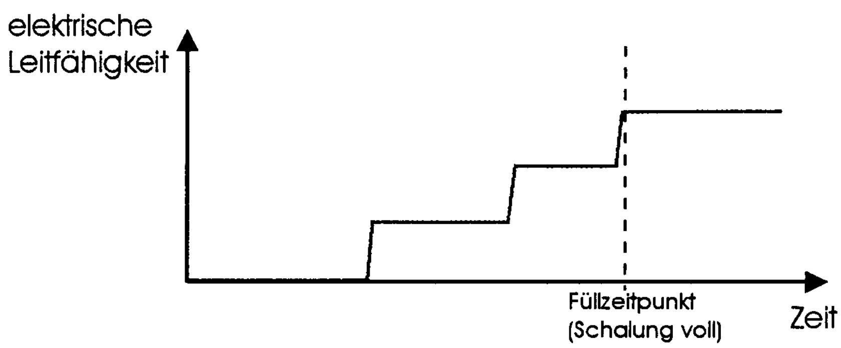


Fig.2b

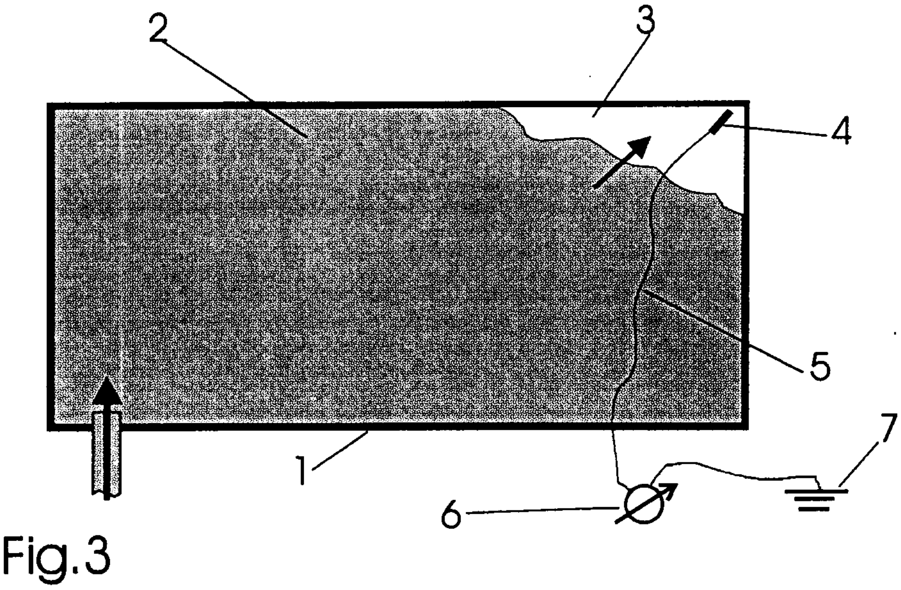


Fig.3

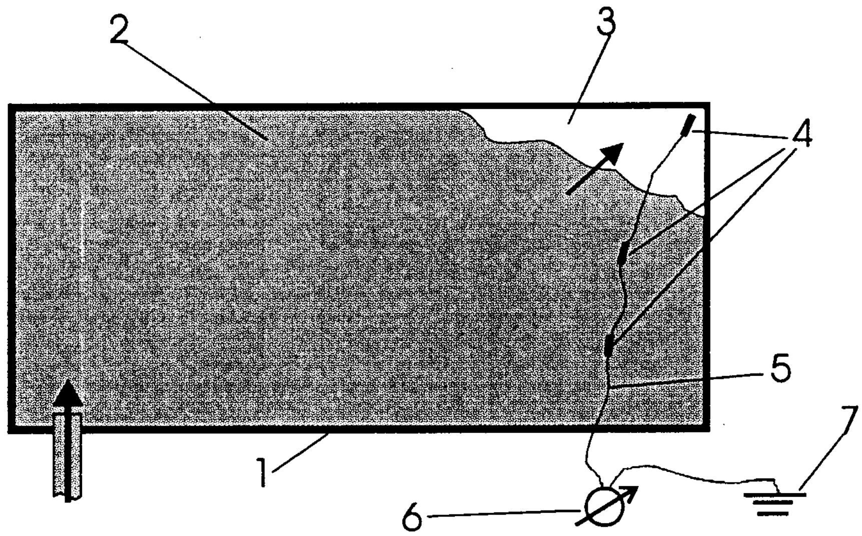


Fig.4

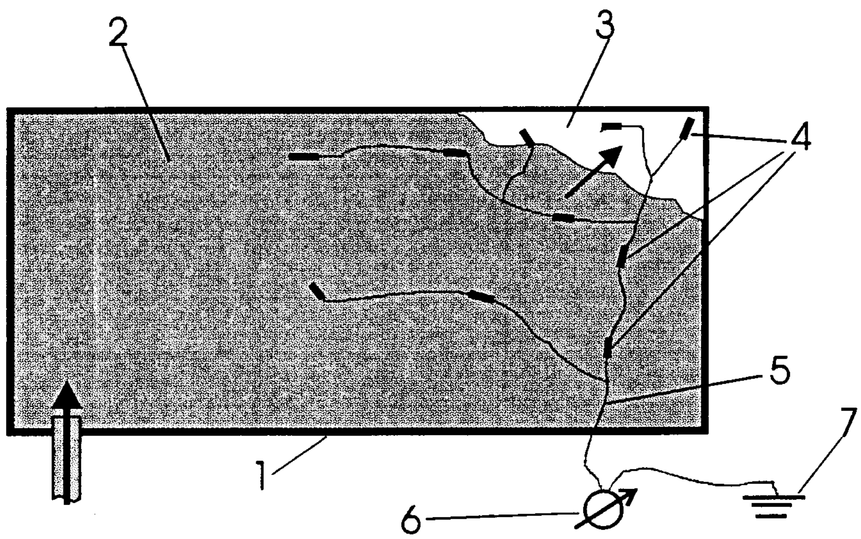


Fig.5

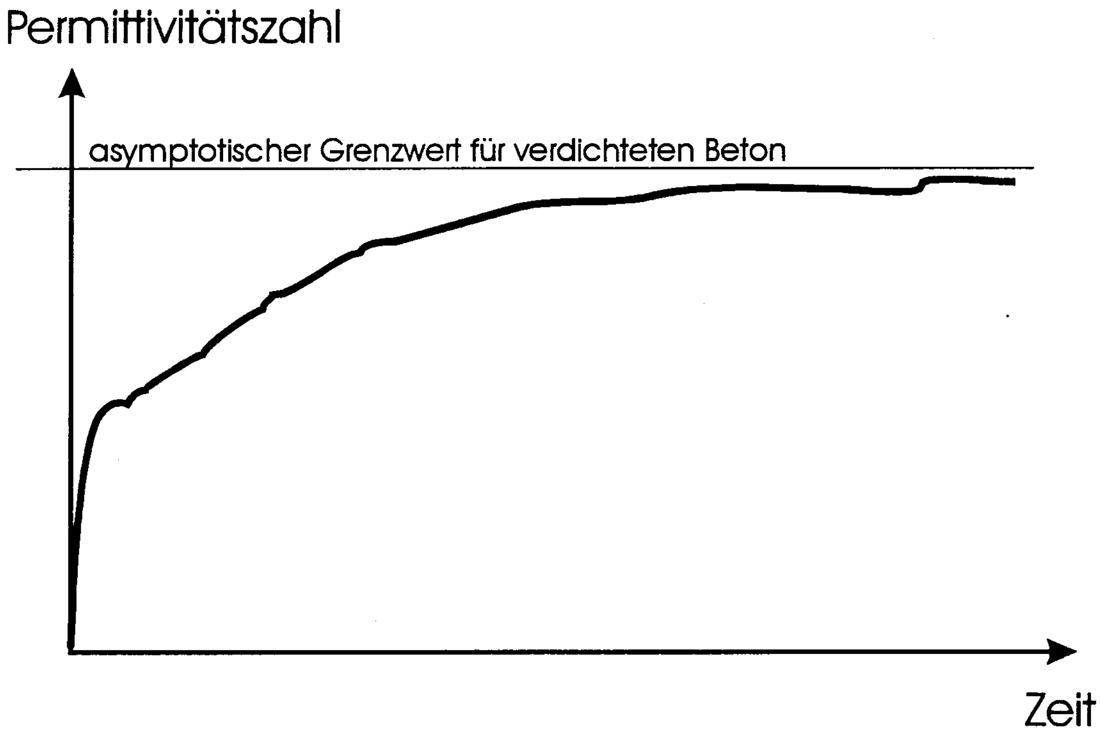
Permittivitätszahl
asymptotischer Grenzwert für verdichteten Beton
Zeit

Fig.6

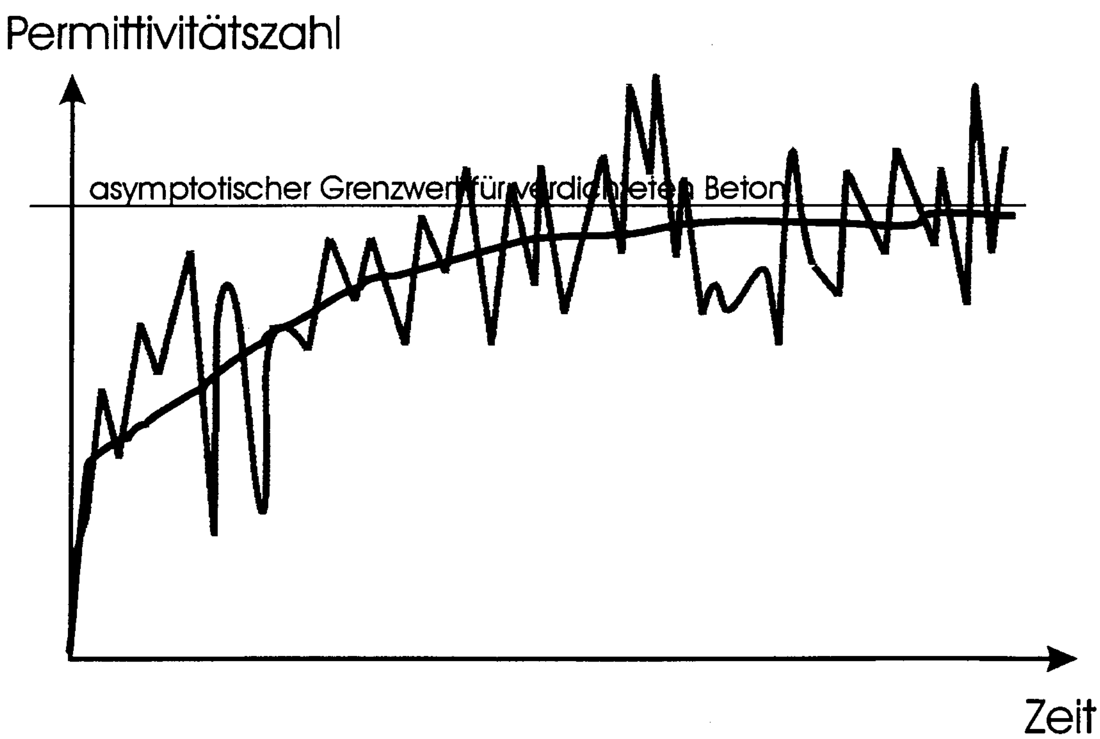
Permittivitätszahl
asymptotischer Grenzwert für verdichteten Beton
Zeit

Fig.7

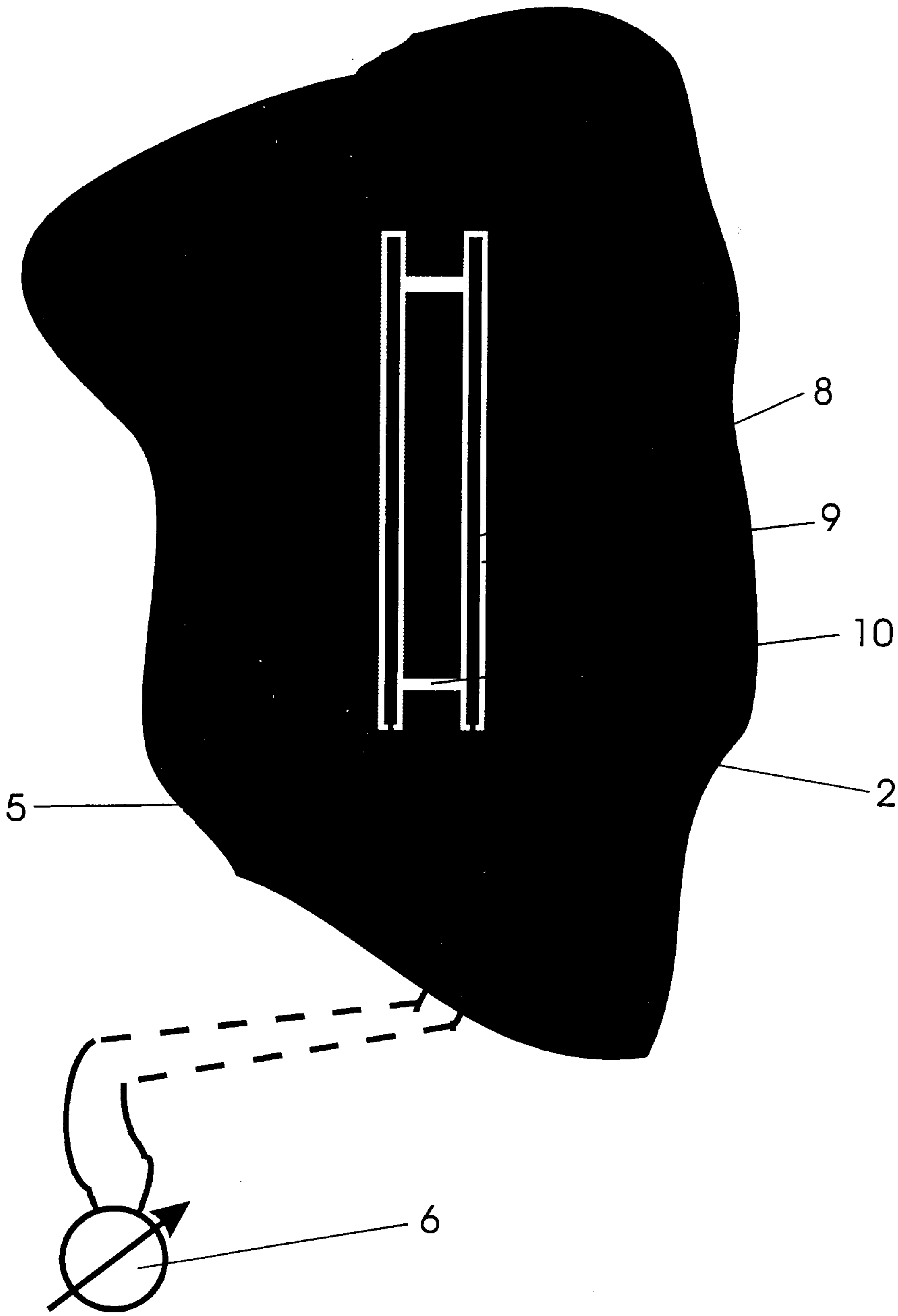


Fig.8